(19) Europäisches Patentamt
European Patent Office
Office européen des brevets

(11) **EP 1 892 001 A1**

(12) **EUROPÄISCHE PATENTANMELDUNG**

(43) Veröffentlichungstag:
 **27.02.2008 Patentblatt 2008/09**

(51) Int Cl.:
 *A61M 1/36* (2006.01)  *A61B 5/103* (2006.01)

(21) Anmeldenummer: 06119406.4

(22) Anmeldetag: **23.08.2006**

(84) Benannte Vertragsstaaten:
 **AT BE BG CH CY CZ DE DK EE ES FI FR GB GR HU IE IS IT LI LT LU LV MC NL PL PT RO SE SI SK TR**
 Benannte Erstreckungsstaaten:
 **AL BA HR MK YU**

(71) Anmelder: **B. Braun Medizintechnologie GmbH 34212 Melsungen (DE)**

(72) Erfinder:
 • **Bock, Gerhard**
  **36289, Friedewald (DE)**

 • **Moll, Stefan**
  **34212, Melsungen (DE)**
 • **Hasberg, Carsten**
  **76135, Karlsruhe (DE)**

(74) Vertreter: **Selting, Günther et al**
 **Von Kreisler Selting Werner**
 **Deichmannhaus am Dom**
 **Bahnhofsvorplatz 1**
 **50667 Köln (DE)**

(54) **Medizinisches Gerät zur extrakorporalen Blutbehandlung**

(57)  Das medizinische Gerät weist Leitungen (22, 23) auf, die mit dem Blutgefäßsystem eines Patienten verbunden werden. Damit ein unbeabsichtigter Blutaustritt entdeckt wird, ist eine Kamera (17) vorgesehen, die auf den extrakorporalen Kreislauf des Patienten gerichtet ist. Die Verarbeitung der Bildsignale der Kamera (17) erfolgt in der Weise, dass die Farbe von Blut und/oder deren Anordnung in der Bildfläche im Kamerabild ermittelt wird, wobei ebenfalls die Größe der aufgenommenen Bildfläche mit der Farbe von Blut ausgewertet wird. Auf diese Weise kann automatisch ein extrakorporaler Blutverlust erkannt und signalisiert werden.

**Fig.2**

EP 1 892 001 A1

**Beschreibung**

Die vorliegende Erfindung betrifft ein medizinisches Gerät zur extrakorporalen Blutbehandlung mit einer Blutbehandlungsvorrichtung, die über extrakorporale Leitungen mit dem Blutkreislauf eines Patienten verbindbar ist, einer Blutpumpe, einer Steuereinheit zum Steuern der Blutpumpe und zur Überwachung von Betriebszuständen und mit einer Kamera zur Aufnahme des extrakorporalen Kreislaufs eines auf einem Behandlungsplatz befindlichen Patienten. Insbesondere betrifft die Erfindung ein medizinisches Gerät, bei dem aufgrund von Undichtigkeiten im extrakorporalen Kreislauf oder von Fehlern am Patientenzugang Blutverlust beim Patienten entstehen kann.

Eine typische Blutbehandlung ist die Dialysebehandlung. Dialyse-Behandlungen werden in der Regel in speziellen Gebäuden durchgeführt. In diesen Gebäuden sind gewöhnlich 20 bis 50 Behandlungsplätze angeordnet, die sich auf mehrere Räume verteilen. Das Pflegepersonal ist für die Überwachung des Patienten zuständig, kann jedoch nicht immer in der Nähe des Patienten sein, da es mehrere Patienten zu betreuen hat. Damit kommt dem Medizingerät die Aufgabe zu, Gefahren für den Patienten zu erkennen, entsprechende Sicherheitssteuerung vorzunehmen und das Pflegepersonal zum Patienten zu rufen. Beim Auftreten eines extrakorporalen Blutverlustes aufgrund einer Diskonnektion im venösen Rücklauf ist die Patienten-Sicherheit zur Zeit nur durch eine sorgfältige Überwachung durch das Pfleger-sonal sichergestellt, da üblich verwendete venöse Drucküberwachung als Schutzsystem, nicht in jedem Fall den Blutverlust erkennt.

Bei der extrakorporalen Blutbehandlung, beispielsweise einer Hämodialyse- oder Plasmabehandlung, strömt das Blut eines Patienten von einem arteriellen Gefäßzugang über einen Filter zu einem venösen Gefäßzugang. Als Zugang zum Blutgefäßsystem wir häufig operativ eine arteriell-venöse Fistel angelegt, die im Allgemeinen mit einer arteriellen und venösen Kanüle punktiert wird. Ebenso ist der Einsatz eines Gefäßimplantats (Shunt) möglich. Unter einem Gefäßzugang wird jede Art des Zugangs zu dem Blutgefäßsystem des Patienten verstanden, insbesondere aber die Verbindung zwischen Arterie und Vene des Patienten. Eine wirksame Sicherheitseinrichtung zur Vermeidung von Blutverlust gibt es bislang bei der Doppelnadel-Behandlung nicht. Zur Zeit behilft man sich lediglich durch Verkleben der Leitungen, die zu oder von einem Gefäßzugang führt, mit Pflastern. Herkömmliche Dialysegeräte messen bei der Rückgabe des Blutes den Widerstand zwischen Gerät und Patient. Dabei wird das Blut mit einer Geschwindigkeit von 200 bis 600 ml/min durch die Kanüle in den Patienten gefördert. Allein der Widerstand der Kanüle liegt dabei größtenteils im Drucküberwachungsbereich des Dialysegerätes. Beim Herausrutschen der venösen Kanüle fließt Blut aus dem arteriellen Gefäßzugang über das Dialysegerät aus dem Patienten. Das Dialysegerät reagiert aufgrund sekundärer Einflüsse wie dem Druckabfall am venösen Druckaufnehmer. Der Druckabfall ist jedoch abhängig vom Blutfluss, Hämatokrit, Kanüle und Gefäßdruck des Patienten. Im Formalfall stellt das Pflegepersonal so nahe wie möglich einen unteren Grenzwert an den aktuellen venösen Druck, ohne genau den Druck zu kennen, der sich bei herausgerutschter Kanüle einstellt. Prinzipiell sind zwei Möglichkeiten vorhanden, nämlich das Dialysegerät gibt Alarm, ohne dass die Kanüle herausgerutscht ist und ruft somit das Pflegepersonal zum Gerät oder das Dialysegerät gibt keinen Alarm, wenn die Kanüle herausgerutscht ist und der Patient verliert Blut.

EP 1 574 178 A1 beschreibt ein medizinisches Behandlungssystem, bei dem eine Videokamera auf den Behandlungsplatz gerichtet ist. Das Bild der Videokamera wird auf dem Bildschirm eines entfernt angeordneten Arztplatzes wiedergegeben. Auf diese Weise kann der Arzt den Patienten visuell sehen bzw. überwachen.

WO 99/24145 A1 beschreibt ein Paar von Elektroden, die in der Nähe der Kanüle angebracht und mit zwei Leitungen mit dem Dialysegerät verbunden sind. Wenn die Nadel herausrutscht, kommt es durch das ausfließende Blut zu einer leitfähigen Verbindung zwischen den Elektroden. Dieses wird durch das Dialysegerät erkannt. Die Steuerung des Dialysegerätes stoppt den Blutfluss und alarmiert das Personal. Dieses Verfahren bedarf zusätzlicher Handhabungen, die vom Personal sorgfältig ausgeführt werden müssen. Außerdem kann des zu Fehlalarmen kommen, wenn zwischen den Elektroden Schweiß sich absetzt.

WO 01/47581 A1 beschreibt eine Anordnung von Elektroden. Damit wird die Erkennung verbessert, in dem ein Generator den Strom kapazitiv zwischen arterieller und venöser Leitung kapazitiv einkoppelt. Bewertet wird der Spannungsabfall, der durch den Stromfluss im Blut in der Leitung verursacht wird. Wenn eine der Kanülen herausrutscht, vermindert sich der Strom, was detektiert wird. Nachteilig bei diesem Verfahren ist, dass eine nicht vollständig herausgerutschte Nadel nicht erkannt wird.

DE 198 48 235 C1 stellt ein System dar, das den arteriellen und venösen Druck bewertet, um daraus das Herausrutschen der venösen Kanüle zu erkennen. Nachteilig bei diesem Verfahren ist, dass das dynamische Verhalten des extrakorporalen Kreislaufs mit eingeht, was zu Fehlbewertungen führen kann. Bei diesem Verfahren ist auch nicht das Problem der indirekten Messung des Blutverlustes gelöst, da der extrakorporale Druck kein Maß für Blutverlust ist.

WO 03/86506 A1 beschreibt einen elektrischen Kontakt, der direkt in das Blut eingebracht wird. Mittels Konstantstrom und Bewertung des Spannungsabfalls wird erkannt, ob die Kanüle herausgerutscht ist. Auch hier ist der Nachteil, dass eine nicht vollständig herausgerutschte Kanüle nicht erkannt wird. Außerdem verteuert sich auch noch das Schlauchsystem, da die elektrischen Kontakte eingebracht werden müssen.

Bei allen Systemen ist keine Erkennung eines Blutverlustes möglich, wenn dieser zum Beispiel an einer Verbindungsstelle im extrakorporalen Schlauchsystem entsteht.

Der Erfindung liegt die Aufgabe zugrunde, ein medizinisches Gerät mit extrakorporalem Blutkreislauf derart auszubilden, dass übermäßiger Blutverlust eines Patienten während der Behandlung mit hoher Sicherheit erkannt wird.

Das medizinische Gerät nach der vorliegenden Erfindung ist durch den Patentanspruch 1 bezeichnet. Es weist eine Farbbildkamera auf, die mit einer Erkennungseinrichtung zur Erkennung der Farbe von Blut und der Größe der von dem Blut eingenommenen Bildfläche verbunden ist. Die Kamera ist auf den Behandlungsplatz bzw. den darauf befindlichen Patienten gerichtet. Sie nimmt Bilder auf, deren Farbinformation von einer Analysiervorrichtung ausgewertet wird. Dabei wird auslaufendes oder ausgelaufenes Blut an seiner typischen Farbe erkannt. Die Menge des ausgelaufenen Blutes wird anhand der Größe der von dem Blut eingenommenen Bildfläche bewertet.

Die von Blut eingenommene Fläche muss nicht zusammenhängend sein sondern kann sich aus mehreren Einzelflächen zusammensetzen.

Die Auswertevorrichtung, an die die Kamera die Bilder liefert, führt folgende Abläufe aus:

- digitale Bilder vom Patienten und dem extrakorporalen Kreislauf aufzunehmen,
- Pixel-Informationen in einen Farbraum transferieren, der die Unterscheidung von Haut zu Blut ermöglicht,
- Pixel als Blut oder nicht Blut klassifizieren,
- Blutpixel summieren,
- die summierten Pixel mit einem Grenzwert vergleichen,
- alternativ Blutflecken feststellen und deren Vergrößerung bewerten,
- die Blutpumpe stoppen und die Schlauchabsperrklemme schließen, wenn der Grenzwert überschritten ist und Alarm geben.

Die Blutzugänge werden normalerweise am Unterarm eines Patienten angelegt. Von dort führen Schläuche zu dem medizinischen Gerät (z. B. Dialysator). Die Blutzugänge werden mit Pflastern an dem Arm des Patienten verklebt. Ein Blutaustritt, der an einem der Gefäßzugänge auftritt, befindet sich in unmittelbarer Nähe des Patientenkörpers, so dass es darauf ankommt, im Kamerabild zwischen Blut und Haut des Patienten zu unterscheiden.

Je nach Menge des Blutverlustes kann dieser tödlich sein. In der Risikobetrachtung wird dies für einen Wert von ca. 500 ml angenommen. Im Einzelnen ist dieser Wert von der persönlichen körperlichen Verfassung des Patienten abhängig.

Vorraussetzung für das sichere Erkennen von fehlerhaften Blutzugängen ist, dass die Blutzugänge für die Kamera sichtbar sind. Dies bedeutet, dass der Patient die Blutzugänge nicht mit einer Decke o. dgl. bedeckt.

Bei einer Digitalkamera wird die Fläche, die mit auslaufendem Blut bedeckt ist, anhand der Pixel ermittelt, die mit der Farbe von Blut belichtet sind. Die Zahl der Blutpixel kann ins Verhältnis gesetzt werden zu der Gesamtzahl der Pixel des Kamerabildes.

Die von einer Digitalkamera erzeugten Bilder liegen in der Regel im RGB-Format (Farbraum) vor. Jeder Bildpunkt (Pixel) setzt sich aus drei Werten zusammen, welche die Farben Rot, Grün und Blau gewichten bzw. in ihrer Intensität darstellen. Jede Gewichtung liegt zwischen 0 und 255. Pro Komponente des Farbmerkmalsvektors ergeben sich 8 Bit bzw. 3 x 8 Bit = 24 Bit pro Bildpunkt. Eine anschauliche Darstellung dieses Farbraumes ist der RGB-Farbwürfel. Dabei sind ungefähr 16 Millionen Farben möglich. Auf der Hauptdiagonalen sind die Grauwerte von schwarz (0, 0, 0) bis weiß (255, 255, 255) zu finden. Weitere Farben sind z. B. rot bei (255, 0, 0) und gelb (255, 255, 0).

Für die Bluterkennung ist das RGB-Format nicht besonders gut geeignet, weil sich konkrete Abgrenzungen nur schwer definieren lassen. Eine bevorzugte Ausgestaltung der Erfindung sieht daher eine Transformationsvorrichtung vor, die im RGB-Farbraum ausgegebene Signale in einen anderen Farbraum transformiert. Dieser andere Farbraum ist vorzugsweise der YUV-Farbraum. Die Transformation bewirkt, dass eine Unterscheidung zwischen Blut- und Nichtblut-Pixeln besser herbeizuführen ist.

Der YUV-Farbraum besteht aus einer Helligkeitskomponente Y und zwei Farbkomponenten U und V. Er geht aus dem RGB-Farbraum durch eine lineare Transformation hervor:

$$\begin{pmatrix} Y \\ U \\ V \end{pmatrix} = \begin{pmatrix} 0.299 & 0.587 & 0.114 \\ -0.148 & -0.289 & 0.437 \\ 0.615 & 0.515 & -0.100 \end{pmatrix} \cdot \begin{pmatrix} R \\ G \\ B \end{pmatrix}$$

Durch die Drehmatrix wird die Hauptdiagonale des RGB-Farbraumes (Grauwerte) auf die Y-Achse des YUV-Farbraumes abgebildet. Ziel ist, eine Trennung von Farb- und Helligkeitsinformation herbeizuführen. Die Wertebereiche liegen für die Y-Komponente bei 0 bis 255 und für die U- und die V-Komponenten bei -128 bis +127.

Die Farbinformation ist in den U- und V-Komponenten gespeichert und die Helligkeitsinformation, die stark von der vorherrschenden Beleuchtung abhängt, in der Y-Komponente.

Beispiele für die Definition der Grenzwerte bzw. Kriterien im YUV-Farbraum werden später beschrieben.

Die Kriterien für die Blut-Diskriminierung hängen von dem Beleuchtungsspektrum der jeweiligen Beleuchtung ab, bei der das Bild von der Kamera aufgenommen wird. So gelten bei Neonbeleuchtung andere Grenzwerte bzw. Kriterien als bei Glühlampenbeleuchtung oder Tageslicht. Zur Feststellung der jeweiligen Beleuchtungsart kann ein Beleuchtungssensor vorgesehen sein, der Mittel zum Auswählen der gespeicherten Kriterien in Abhängigkeit von dem detektierten Beleuchtungsspektrum steuert.

Alternativ besteht die Möglichkeit, die Beleuchtung mit einem definierten Beleuchtungsspektrum vorzunehmen und für die Aufnahme der Kamerabilder eine eigene definierte Beleuchtungsquelle einzusetzen. Diese kann beispielsweise aus einem Blitzlichtgerät bestehen, dessen Beleuchtungsstärke so groß ist, dass sie Fremdlicht überdeckt, so dass die Auswertung des Farbspektrums eines Pixel-Bildes auf der Basis des Beleuchtungsspektrums der definierten Beleuchtungsquelle erfolgen kann.

Die Erfindung ermöglicht es auch, die Ausbreitungsgeschwindigkeit eines Blutflecks mit der Kamera zu erfassen, indem die Vergrößerung der Fläche mit der Farbe von Blut innerhalb einer definierten Zeit erfasst bzw. berechnet wird. Diese Art der Auswertung kann auch in der Weise erfolgen, dass bei hoher Ausbreitungsgeschwindigkeit des Blutes die Bildfolgefrequenz der Kamera automatisch erhöht wird.

Der Blutverlust kann auch dadurch festgestellt werden, dass eine Einrichtung zum Summieren der Bild-Pixel oder einer Fläche mit der Farbe von Blut über mehrere Bilder der Kamera hinweg zur Ermittlung des Blutverlustes vorgesehen ist. Dabei kann auch der Umriss der von Blut eingenommenen Fläche (oder mehrerer Flächen) herangezogen werden.

Damit die Kameraüberwachung auf das Auslaufen von Blut erst dann durchgeführt wird, wenn der Patient an das extrakorporale Schlauchsystem angeschlossen ist und das Blut in diesen strömt, kann in dem extrakorporalen Leitungssystem ein Blutdetektor vorgesehen sein, der nur im Falle einer Erkennung von Blut die Aktivierung der Kamera zulässt. Dieser Blutdetektor ist beispielsweise ein Rot-Detektor, der eine rote Flüssigkeit in dem Schlauchsystem erkennt.

Damit die Kamera das richtige Zielgebiet aufnimmt, kann sie einen Antrieb zur Selbstausrichtung aufweisen, der die Kamera auf eine Referenzmarkierung ausrichtet. Diese Referenzmarkierung, beispielsweise ein Tag oder ein Button, wird auf dem Zielgebiet befestigt, beispielsweise auf dem Unterarm des Patienten. Wenn die Referenzmarkierung nicht gefunden wird, erfolgt eine Alarmgabe.

Die Übertragung der Bilder von der Kamera zu dem Gerät erfolgt über Kabel oder über eine drahtlose Verbindung. Es besteht auch die Möglichkeit, mehrere Kameras vorzusehen, die aus unterschiedlichen Blickrichtungen das Zielgebiet aufnehmen.

Das Gerät weist vorzugsweise eine Lesevorrichtung auf, die eine Identifikation des Bedienungspersonals liest, beispielsweise eine maschinenlesbare Personalkarte. Die Daten dieser Identifikation werden gespeichert, so dass dokumentiert wird, welche Person an dem Gerät zu welcher Zeit hantiert hat und welche Einstellungen vorgenommen wurden.

Das Einlesen der Identifikation gibt auch Aufschluss darüber, dass eine Pflegeperson an dem Behandlungsplatz anwesend ist. Dies kann für die Auswertung der Signale, die den Blutverlust angeben, herangezogen werden. So kann beispielsweise die bisherige Blutfläche als Referenzgröße herangezogen werden, so dass nur Veränderungen, die seit dem Erscheinen der Behandlungsperson aufgetreten sind, registriert werden.

Das erfindungsgemäße Verfahren kann auch zur Überwachung extrakorporaler Blutkreisläufe auf Undichtigkeit angewendet werden, bei denen keine Blutbehandlung erfolgt. Beispiele hierfür sind eine Blutabnahme und ein Blutkreislaufsystem während einer Operation.

Im Folgenden werden unter Bezugnahme auf die Zeichnungen Ausführungsbeispiele der Erfindung näher erläutert.

Es zeigen:

Fig. 1     eine perspektivische Ansicht des medizinischen Gerätes in Form eines Gerätes für die Hämodialyse, Hämofiltration oder Plasmabehandlung,

Fig. 2     eine schematische Darstellung der wesentlichen Funktionsteile eines Dialysegerätes,

Fig. 3     die Darstellung eines Armes eines Patienten mit den Gefäßzugängen, wie von der Kamera aufgenommen,

Fig. 4-6   Histogramme der Farbe von Blut im YUV-Farbraum mit Beispielen für Grenzwerte, die die Kriterien für die Bluterkennung bilden, wobei U und V um +128 verschoben sind,

Fig. 7       ein Beispiel einer Pixelverteilung in der UV-Ebene mit Beispielen für Grenzwerte,

Fig. 8       ein Diagramm der Häufigkeiten über der Helligkeitskomponente Y im YUV-Farbraum für die Bewertung von Blut und Haut.

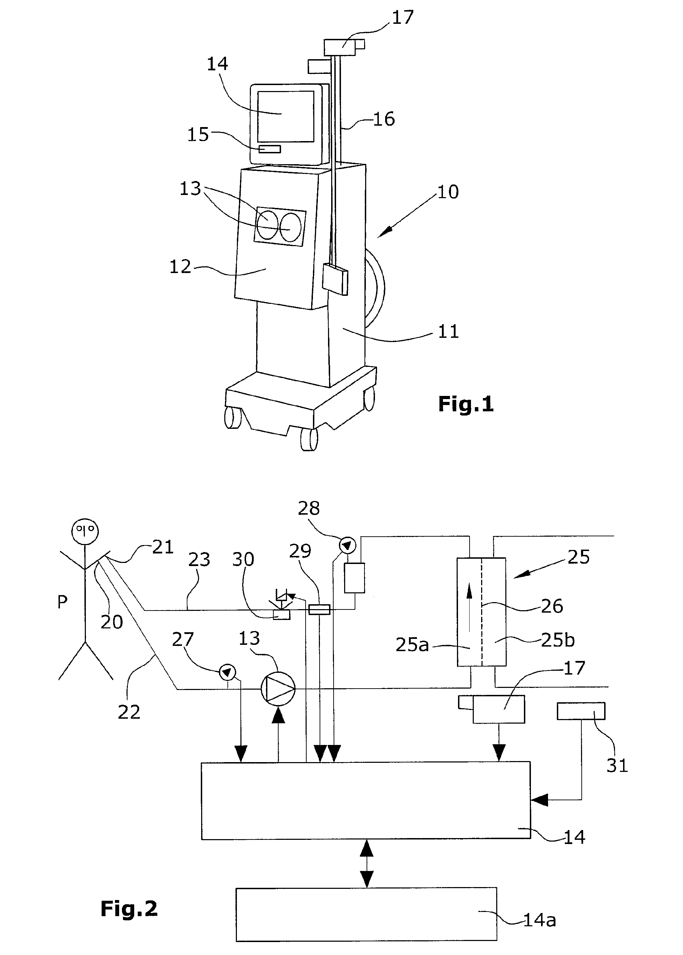

Figur 1 zeigt ein medizinisches Gerät 10 zur extrakorporalen Blutbehandlung. Dieses Gerät weist einen Gerätesockel 11 auf, in dem sich die mechanischen Komponenten befinden und der an seiner Vorderseite eine Konsole 12 trägt, in der zwei von außen zugängliche Blutpumpen 13 angeordnet sind. Die Blutpumpen sind Schlauchpumpen, deren Schläuche von der Frontseite her eingelegt werden.

Auf dem Gerätesockel 11 befindet sich die Steuereinheit 14, welche auch das Interface für die Kommunikation mit dem Benutzer bildet. Die Steuereinheit 14 weist hier einen Touchscreenmonitor auf, über den der Benutzer verschiedene Menüs aufrufen und Betriebszustände abfragen sowie Daten und Befehle eingeben kann. Ein Kartenleser 15 ist vorgesehen, in den der Benutzer eine maschinenlesbare Identifikationskarte einführen kann.

An einer Infusionsstange 16 ist eine Kamera 17 befestigt. Hierbei handelt es sich um eine digitale Farbbildkamera. Die von der Kamera 17 aufgenommenen Einzelbilder werden an die Steuereinheit 14 übertragen. Die Kamera 17 ist auf einen Patientenplatz gerichtet, z. B. eine Liege, auf der der Patient während der Blutbehandlung liegt. Auf diese Weise wird der extrakorporale Kreislauf von dem Kamerabild erfasst.

Der Patientenkörper wird über Schläuche an das Gerät 10 angeschlossen.

In Figur 2 ist ein Patient P schematisch dargestellt. Ein Arm des Patienten ist mit einem arteriellen Zugang 20 und einem venösen Zugang 21 versehen. Von dem arteriellen Zugang 20 führt eine arterielle Schlauchleitung 22 zu der Blutpumpe 13. Diese pumpt das Blut durch die Blutkammer 25a einer Behandlungsvorrichtung 25 in Form eines Dialysators, dessen beide Kammern 25a, 25b durch eine Membran 26 getrennt sind. Die Kammer 25b ist eine Dialysierflüssigkeitskammer, die von Dialysierflüssigkeit durchflossen wird.

Nach Verlassen der Kammer 25a strömt das Blut in eine venöse Schlauchleitung 23, die mit dem venösen Gefäßanschluss 21 verbunden ist. Auf diese Weise wird ein Blutkreislauf gebildet.

Die arterielle Leitung 22 enthält einen Druckmesser 27 zur Messung des arteriellen Blutdrucks. In gleicher Weise enthält die venöse Leitung einen Druckmesser 28 zur Messung des venösen Blutdrucks. Ferner enthält die venöse Leitung 23 einen Rot-Detektor 29, der die Anwesenheit von Blut in der Schlauchleitung erkennt und an die Steuereinheit 14 meldet. Auch die Druckmesser 27, 28 sind mit der Steuereinheit 14 verbunden. Diese steuert den gesamten Betrieb des Gerätes und überwacht die beschriebenen Funktionen sowie eine Reihe anderer Funktionen, die hier nicht näher erläutert werden.

Um den Blutkreislauf absperren zu können, ist in der venösen Leitung 23 eine von der Steuereinheit 14 gesteuerte Schlauchabsperrklemme 30 vorgesehen. Auch in der arteriellen Leitung 22 befindet sich ein Absperrorgan in Form der Blutpumpe 13. Die Blutpumpe ist eine Schlauchpumpe, die von einem Quetschorgan fortlaufend abgequetscht wird. Bei Stillstand der Blutpumpe wirkt diese als Absperrorgan, dass die Schlauchleitung verschließt.

Die Kamera 17 und ihr Anschluss an die Steuereinheit 14 sind in Figur 2 erkennbar. Außerdem ist ein Beleuchtungssensor 31 vorgesehen, der den Typ des Beleuchtungslichtes erkennt, beispielsweise Neonlicht, Licht mit UV-Lichtanteil oder warmes Leuchtlicht. In Abhängigkeit davon werden die Kriterien für die Bluterkennung verändert.

Figur 3 zeigt das von der Kamera 17 aufgenommene Bild des zu beobachtenden Bereichs. Am Arm des Patienten, der auf einem Behandlungsplatz liegt, sind die Gefäßzugänge 20,21 angebracht und von diesen führen die Schlauchleitungen 22, 23 zu der Blutpumpe. Am Körper des Patienten, hier am Arm, ist eine Referenzmarkierung 35 angebracht, die von der Kamera 17 detektierbar ist. Die Kamera 17 wird von einem (nicht dargestellten) Bewegungsantrieb so eingestellt, dass die Referenzmarkierung 35 sich an einer bestimmten Stelle im Kamerabild befindet. Auf diese Weise wird sichergestellt, dass unabhängig von Bewegungen des Patienten die Kamera stets auf das gewünschte Zielgebiet gerichtet ist.

Die Steuereinheit 14 in Figur 2 ist ein Computer mit Speichereinheit. Sie führt auch sämtliche Überwachungs- und Steuerabläufe durch, sowie die Alarmerzeugungen. Die Steuereinheit 14 ist mit einer Anzeige-, Bedien- und Kommunikationseinheit 14a verbunden.

Bei der Dialyse wird bei einem Patienten mit der Blutpumpe ein extrakorporaler Blutfluss von 50 bis 600 ml/min aufgebaut, indem das Blut mittels einer Schlauchpumpe aus der arteriellen Kanüle gezogen und über die venöse Kanüle zurückgegeben wird. Das Blut wird in Leitungen geführt, die an die Komponenten, wie Kanülen, Druckaufnehmer und Dialysator, angeschlossen werden. Die Steuerung und Überwachung geschieht mittels einer Steuer-, Rechen- und Speichereinheit. Die Parameter führ den zu behandelten Patienten werden über die Anzeige-, Bedien- und Kommunikationseinheit eingegeben. Für die Unterbrechung des Blutflusses stoppt die Steuereinheit die Blutpumpe und schließt de Schlauchabsperrklemme. Außerdem wird ein optischer und akustischer Alarm ausgelöst. Damit wird der Patient vor weiterem Schaden geschützt, weil es zu keinem weiteren Blutverlust kommen kann.

Die von der Digitalkamera erzeugten Bilder liegen in der Regel im RGB-Format vor. Jeder einzelne Bildpunkt setzt sich aus drei Werten zusammen, welche die Farben Rot, Grün und Blau gewichten. Jede Gewichtung liegt zwischen 0 und 255. Die im RGB-Farbraum vorliegenden Bildinhalte werden durch eine Transformationsvorrichtung in einen anderen Farbraum transformiert. Hierbei handelt es sich vorzugsweise um den YUV-Farbraum, der für die Bluterkennung besser geeignet ist. Ziel der Transformierung ist es, die Unterscheidung zwischen Blut- und Nichtblut-Pixeln herbeizuführen. Außer dem YUV-Farbraum können auch andere Farbräume wie z. B. HSV und Lab benutzt werden.

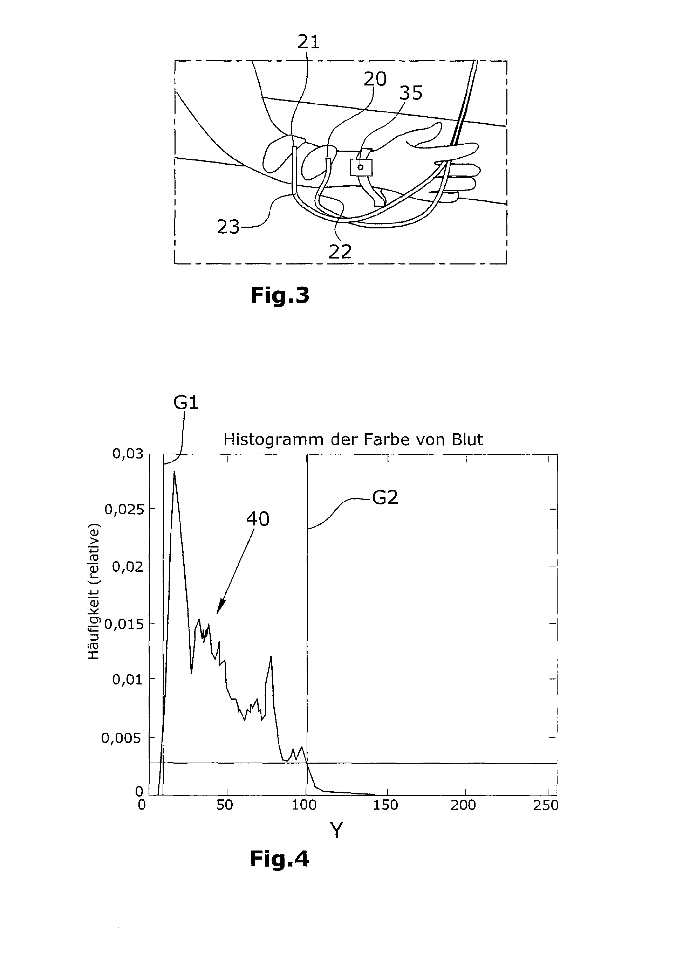

Für die Abgrenzung zwischen Pixeln der Farbe von Blut und der Farbe von Nichtblut werden scharfe Grenzen in dem Farbraum eingeführt. Liegt das zu untersuchende Pixel innerhalb des durch die Grenzen markierten Unterraumes, wird es als Blutpixel klassifiziert. Die Grenzen oder Kriterien der Komponenten Y, U und V ergeben sich aufgrund von Lerndaten, die beim Aufnehmen von Blut unter verschiedenen Beleuchtungsspektren aufgenommen werden. In den Figuren 4, 5 und 6 sind verschiedene aufgenommene Histogramme bei einer bestimmten Beleuchtung dargestellt. Hierbei ist entlang der Abszisse jeweils der Wert Y, U und V aufgetragen und entlang der Ordinate die relative Häufigkeit. Die U- und die V-Achse sind jeweils um +128 verschoben.

Die Kurve 40 in Figur 4 zeigt die Häufigkeitsverteilung des Helligkeitswertes Y für Blut. Man erkennt, dass Blut bei Werten von Y zwischen G1=10 und G2=100 vorliegen kann.

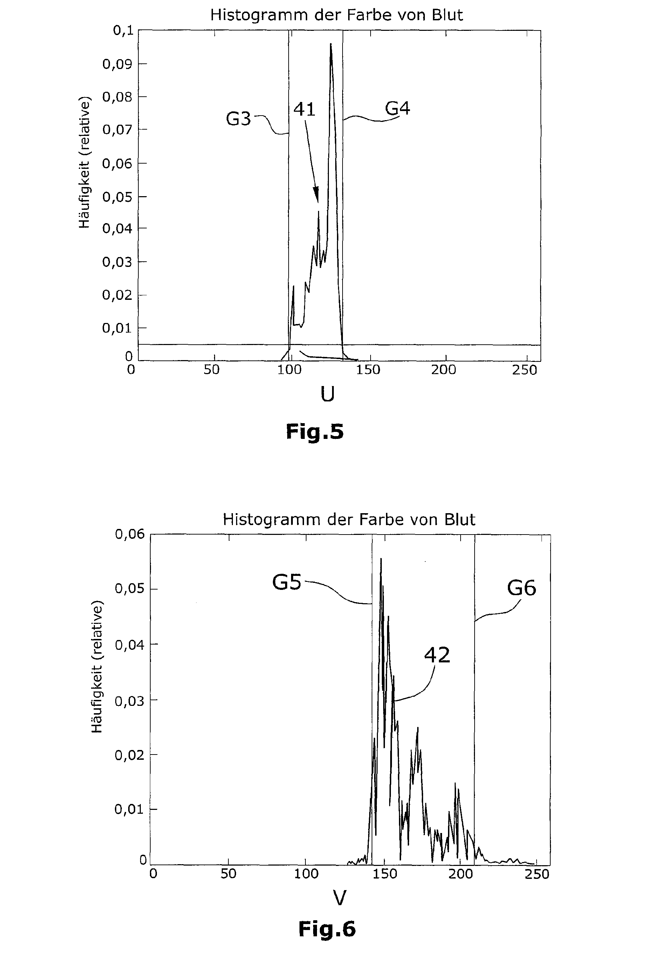

Figur 5 zeigt die Häufigkeitsverteilung 41 für den Wert U bei Blut. Die Kurve 41 liegt zwischen den Grenzen G3 = 96 und G4 = etwa 130.

Figur 6 zeigt die Häufigkeitsverteilung 42 für V von G5=140 bis G6=210.

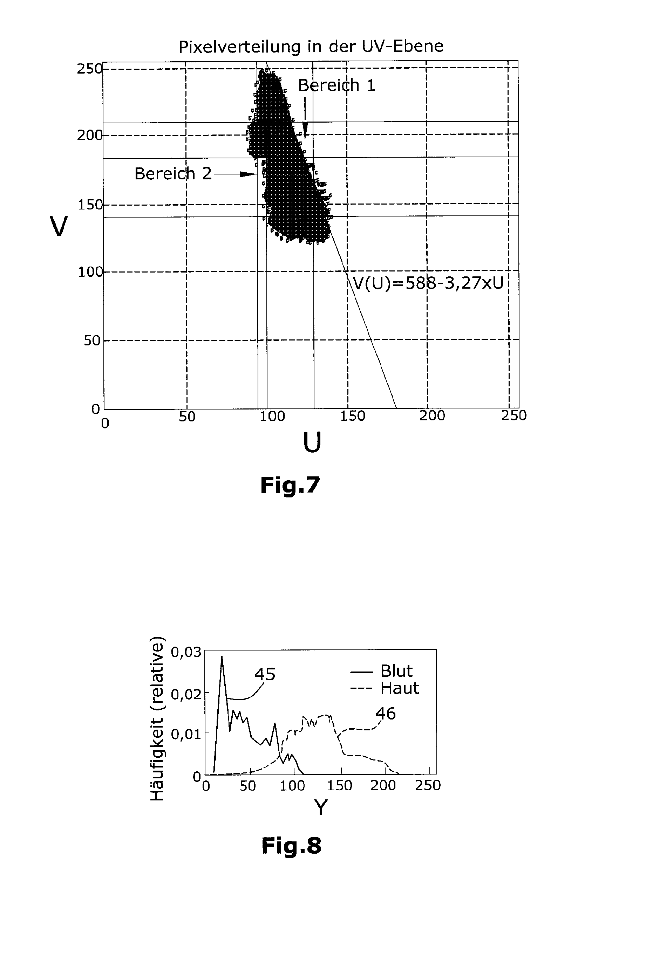

Figur 7 zeigt die Pixelverteilung von Blut in der UV-Ebene.

Die größte Störgröße bei der Unterscheidung, ob ein Pixel Blut darstellt oder nicht, ist die Haut. Um diese zu eliminieren hat sich gezeigt, dass der YUV Farbraum mit einer scharfen Grenze bei der Y-Komponente geeignet ist, Blut und Haut zu unterscheiden.

Figur 8 zeigt die Häufigkeitsverteilung der Komponente Y, wobei die Kurve 45 Blut und die Kurve 46 Hautfarbe bedeutet. Beide lassen sich bis auf einen Überschneidungsbereich gut voneinander unterscheiden.

Für die Bewertung der Pixel können nachfolgende Modellierungen angewandt werden:

- Explizit YUV

    P1:     Y_[10, 90]
    P2:     U_[96, (588 - V)/3.27]
    P3:     U_[102, (588 - V)/3.27]
    P4:     V_[184, 242]
    P5:     V_[140, 184]
    R:      Wenn [P1 und [(P2 und P4) oder (P3 und P5)]] Dann [Pixel = Blut] Bei der obigen Schreibweise besteht der Parameter P1 darin, dass Y zwischen 10 und 90 liegt. Die Regel R gibt diejenigen Bedingungen an, die erfüllt sein müssen, damit ein Pixel des Kamerabildes als Blut erkannt wird, und bedeutet eine Bool'sche logische Verknüpfung.

- Nichtparametrisch UV/Explizit Y
  Dieser Ansatz kombiniert die nichtparametrische Modellierung der UV-Komponenten mit einer expliziten Modellierung der Y-Komponente. Die Auswertung des nichtparametrischen Modells erfolgt über einen Schwellwert. Übersteigt der Wert einen vorgegebenen Grenzwert und ist Y_[10, 90] erfüllt, wird der Bildpunkt als Blutpixel klassifiziert.

- Parametrisch UV/Explizit Y
  Dieser Ansatz kombiniert die parametrische Modellierung der UV-Komponenten mit einer expliziten Modellierung der Y-Komponente. Die Auswertung des parametrischen Modells erfolgt über den Mahalanobis-Distanz. Liegt der berechnete Abstand unterhalb eines vorgegebenen Schwellwertes und ist Y_[10, 90] erfüllt, wird der Bildpunkt als Blutpixel klassifiziert.

- Parametrisch YUV
  In diesem Ansatz werden alle drei Farbraumkomponenten mit Hilfe einer Gaußverteilung modelliert. Für die Parameter der Gaußverteilung werden die Lerndaten herangezogen. Die Auswertung erfolgt durch den Mahalanodisabstand. Ist der Abstand zwischen Bildpunkt und Gaußverteilung kleiner als ein Schwellwert, wird dieser Bildpunkt als Blutpixel klassifiziert.

Gleiche oder ähnliche Regeln können auch für die anderen Farbräume aufgestellt werden, wobei das Prinzip der Lerndaten (Referenz) herangezogen wird. Außerdem ist es möglich, zur Bildaufnahme ein spezifisches Licht (zwischen Infrarot und Ultraviolett) zur Bildaufnahme von der Kamera auszusenden.

Für die weitere Verwertung der gewonnenen Blutpixel sind zwei Lösungen anwendbar. Beiden Lösungen unterliegt das Prinzip der Bildfolge. Das heißt, dass Bilder in einem Abstand von einigen Sekunden aufgenommen werden und dass das aktuelle Bild seine Bewertung aus der Historie bezieht. Mit dieser Methode werden die Blutpixel als Summe oder als Fläche als Maß für den Blutverlust herangezogen. Um Fehlalarme zu vermeiden, können Referenzmarkierungen, die von der Kamera oder einem Laserstrahl abgetastet werden, angebracht werden, auf die sich die Kamera fokussieren kann.

Im weiteren geht es darum, mit den klassifizierten Blutpixeln in der Bilderabfolge eine Entscheidung zu fällen, ob ein extrakorporaler Blutaustritt vorliegt oder nicht.

Hierbei werden folgende Schritte ausgeführt:

1. Aufnahme des Bildes
2. Klassifikation der Blutpixel im Bild
3. Auswertung der Bildfolge der klassifizierten Blutpixel

Der dritte Schritt mündet in die Entscheidung, ob der kritische Blutverlust erreicht ist oder nicht. Mit der nachfolgenden Beziehung wird die maximale Zeit zwischen zwei Bildern ermittelt:

$$\Delta t = \frac{Q_{Blut\_extrakorporal}}{V_{Blut\_kritisch}}$$

Aus dem Zusammenhang zwischen dem Volumen des ausgetretenen Blutes $V_{Blut}$ und der daraus entstandenen Blutfläche $A_{Blut}$, gibt es einen Faktor x, der die Ausbreitungsgeschwindigkeit der Blutfläche darstellt. Der Faktor x für die Ausbreitungsgeschwindigkeit, Fläche pro Volumen, ist von der Beschaffenheit der Oberfläche, auf der sich das Blut ausbreitet, abhängig und wird aus einer Tabelle entnommen:

$$x = \frac{A_{Blut}}{V_{Blut}}$$

Die Pixel im Bild, die einen Blutfleck abbilden, werden über den Zusammenhang Oberfläche des aufgenommenen Bildausschnitts und Auflösung der Kamera in Bezug gebracht. Bei einer Kamera mit einer Pixelanzahl $N_{Bild}$ ergibt sich der nachfolgende Zusammenhang für die Blutpixelanzahl:

$$N_{Blut(t)} = \frac{N_{Bild}}{A_{Bild}} \cdot x \cdot Q_{Blutverlust} \cdot t$$

Hierin ist $N_{Blut(t)}$ die Pixelzahl der Farbe von Blut, $A_{Bild}$ die Bildfläche und $Q_{Blutverlust}$ der ausgetretene Blutstrom, t ist die Zeit.

Zwischen zwei Bildern ergibt sich der Blutverlust $\Delta V$ aus der nachfolgenden Berechnung:

$$\Delta V_{Blutverlust(k)} = \left( N_{Blut(k)} - N_{Blut(k-1)} \right) \cdot \frac{A_{Bild}}{N_{Bild} \cdot x}$$

k ist die laufende Nummer eines Bildes.

Dabei wird zu Grunde gelegt, dass vor dem Anlegen des Patienten an den extrakorporalen Kreislauf kein Blutverlust vorhanden ist. Das Starten der Kamera beziehungsweise die Bewertung der Bilder erfolgt manuell und/oder durch Sensoren und/oder durch Betriebszustände, die extrakorporales Blutfluss annehmen lassen. Der Blutverlust ergibt sich aus der Summe der Deltavolumen bis zum ersten Bild oder bis zu dem Bild, bei dem die Anwesenheit des Bedienpersonal detektiert wird. Das Erkennen von dem Bediener geschieht mittels Bilderkennung durch die Kamera, Identifikation durch Chipkarte, Tastatureingabe usw.

Der extrakorporale Blutkreislauf wird gestoppt und ein Alarm ausgelöst, wenn die Summe des Blutverlustes größer als ein festgelegter Grenzwert ist.

Abnormale Zustände wie

a) kurzfristige Abdeckung des zu überwachenden Bildausschnitts
b) Patient bewegt sich und damit eine Änderung des Bildausschnitts
c) Auftauchen von blutähnlichen Flecken (Zeitschriften, Kleidungsstücke)

können durch logische Vergleiche während der Bildauswertung wie nachfolgend beschrieben behandelt werden, um Fehlalarme zu vermeiden.

1. Sollte die Anzahl der Blutpixel deutlich sinken, so wird ein Hinweis gegeben, der das Personal auffordert, die Kamera neu auszurichten (Fall a).

2. Negativer Blutverlust (weniger Blutpixel als das Startbild) wird ignoriert oder als Startbild gesetzt (Fall b).

3. Ergibt sich ein höherer Blutverlust (Blutpixel) als extrakorporal gefördert, so wird dies ignoriert und ein Hinweis ausgegeben, der das Personal zu korrigierenden Maßnahmen auffordert (Fall c).

4. Beim Fall b kann über eine motorische Kamera kompensiert werden, indem eine Bilderkennung oder Markierung die Kamera ausrichtet.

5. Liegen einzelne Blutpixel nicht in einer Flächenanordnung, so werden diese für die Auswertung ignoriert.

**Patentansprüche**

1. Medizinisches Gerät zur extrakorporalen Blutbehandlung, mit einer Behandlungsvorrichtung (25), die über extrakorporale Leitungen (22, 23) mit dem Blutkreislauf eines Patienten verbindbar ist, mindestens einer Blutpumpe (13), einer Steuereinheit (14) zum Steuern der Blutpumpe und zur Überwachung von Betriebszuständen, und einer Kamera (17), die auf einen Behandlungsplatz richtbar ist,
**dadurch gekennzeichnet,**
**dass** die Kamera (17) eine Farbbildkamera ist, die mit einer Erkennungseinrichtung zur Erkennung der Farbe von Blut und der Größe der von dem Blut eingenommen Bildfläche verbunden ist.

2. Medizinisches Gerät nach Anspruch 1, **dadurch gekennzeichnet, dass** die Kamera (17) eine Digitalkamera ist und dass Mittel vorgesehen sind, um die Anzahl der Pixel zu ermitteln, die mit der Farbe von Blut belichtet sind.

3. Medizinisches Gerät nach Anspruch 1 oder 2, **dadurch gekennzeichnet, dass** eine Transformationsvorrichtung vorgesehen ist, die die im RGB-Farbraum ausgegebenen Signale der Kamera in einen anderen Farbraum transformieren.

4. Medizinisches Gerät nach Anspruch 3, **dadurch gekennzeichnet, dass** der andere Farbraum der YUV-Farbraum ist.

5. Medizinisches Gerät nach einem der Ansprüche 1-4, **gekennzeichnet durch** eine Speichereinrichtung zur Speicherung von Kriterien für die Bluterkennung.

6. Medizinisches Gerät nach Anspruch 5, **dadurch gekennzeichnet, dass** die Speichereinrichtung unterschiedliche Kriterien für unterschiedliche Beleuchtungsspektren enthält.

7. Medizinisches Gerät nach Anspruch 5 oder 6, **gekennzeichnet durch** einen Beleuchtungssensor (31) zum Detektieren des Beleuchtungsspektrums und Mittel zum Auswählen der gespeicherten Kriterien in Abhängigkeit von dem detektierten Beleuchtungsspektrum.

8. Medizinisches Gerät nach einem der Ansprüche 1-7, **dadurch gekennzeichnet, dass** eine Beleuchtungsvorrichtung zur Beleuchtung des Aufnahmegebietes der Kamera (17) mit Licht von vorbestimmtem Beleuchtungsspektrum vorgesehen ist.

9. Medizinisches Gerät nach einem der Ansprüche 1 - 8, **gekennzeichnet durch** eine Einrichtung zur Berechnung der Geschwindigkeit der Vergrößerung der Fläche mit der Farbe von Blut.

10. Medizinisches Gerät nach Anspruch 9, **dadurch gekennzeichnet, dass** die Bildfolgefrequenz der Kamera (17) entsprechend der Geschwindigkeit der Vergrößerung der Fläche von Blut gesteuert ist.

11. Medizinisches Gerät nach einem der Ansprüche 1 - 10, **dadurch gekennzeichnet, dass** eine Einrichtung zum Summieren der Bildpixel mit der Farbe von Blut über mehrere Bilder der Kamera hinweg zur Ermittlung des Blutverlustes vorgesehen ist.

12. Medizinisches Gerät nach einem der Ansprüche 1 - 11, **dadurch gekennzeichnet, dass** die Steuereinheit (14) Absperrorgane (13, 30) in den extrakorporalen Blutkreislauf aktiviert, wenn ein Grenzwert der Fläche mit der Farbe von Blut oder ein Grenzwert der Geschwindigkeit der Vergrößerung dieser Fläche überschritten wird.

13. Medizinisches Gerät nach einem der Ansprüche 1 - 12, **dadurch gekennzeichnet, dass** in dem extrakorporalen Leitungssystem ein Blutdetektor (29) vorgesehen ist, der im Fall einer Erkennung von Blut die Aktivierung der Kamera (17) zulässt.

14. Medizinisches Gerät nach einem der Ansprüche 1 - 13, **dadurch gekennzeichnet, dass** die Kamera (17) einen Antrieb zur Selbstausrichtung aufweist und sich auf eine Referenzmarkierung (35) ausrichtet.

15. Medizinisches Gerät nach Anspruch 14, **dadurch gekennzeichnet, dass** eine Alarmerzeugung erfolgt, wenn die Kamera (17) die Referenzmarkierung (35) nicht findet.

16. Verfahren zur Überwachung des Betriebes eines medizinischen Gerätes zur extrakorporalen Blutbehandlung, bei welchem eine Kamera einen auf einem Behandlungsplatz befindlichen Patienten aufnimmt, **dadurch gekennzeichnet, dass** mindestens eine Farbbildkamera verwendet wird, deren Farbsignale zur Erkennung der Farbe von Blut ausgewertet werden und dass aus den Farbsignalen die Größe der von dem Blut eingenommenen Bildfläche ermittelt wird.

17. Verfahren nach Anspruch 16, **gekennzeichnet durch** die Verwendung einer Digitalkamera und die Ermittlung der Anzahl der Pixel, die mit der Farbe von Blut belichtet sind.

18. Verfahren nach Anspruch 16 oder 17, **dadurch gekennzeichnet, dass** die im RGB-Farbraum ausgegebenen Bildsignale der Kamera in einen anderen Farbraum transformiert werden.

**Fig.1**

**Fig.2**

**Fig.3**

**Fig.4**

## Histogramm der Farbe von Blut

**Fig.5**

## Histogramm der Farbe von Blut

**Fig.6**

## Pixelverteilung in der UV-Ebene

**Fig.7**

**Fig.8**

**Europäisches Patentamt**

## EUROPÄISCHER RECHERCHENBERICHT

Nummer der Anmeldung

EP 06 11 9406

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | EP 1 574 178 A2 (BRAUN MEDIZINTECHNOLOGIE GMBH [DE]) 14. September 2005 (2005-09-14) * Absätze [0007], [0013], [0022], [0023] * * Abbildung 1 * ----- | 1-18 | INV. A61M1/36 A61B5/103 |
| A | DE 40 14 572 A1 (STAEBLEIN ALEXANDER [DE]) 14. November 1991 (1991-11-14) * Spalte 2, Zeile 1 - Spalte 3, Zeile 1 * * Spalte 5, Zeilen 30-35 * * Abbildungen 1,4 * ----- | 1,16 | |
| A | US 2003/194894 A1 (WARIAR RAMESH [US] ET AL) 16. Oktober 2003 (2003-10-16) * Absätze [0014], [0017], [0043], [0048], [0049] * * Abbildungen 1C,3,4B * ----- | 1,16 | |
| A | DE 198 48 235 C1 (FRESENIUS MEDICAL CARE DE GMBH [DE]) 16. März 2000 (2000-03-16) * Seite 2, Zeilen 45-63 * * Seite 4, Zeilen 16-24 * ----- | 1,16 | RECHERCHIERTE SACHGEBIETE (IPC) |
| A | WO 2004/074822 A (LEICA MICROSYSTEMS SEMICONDUCT [DE]; MICHELSSON DETLEF [DE]) 2. September 2004 (2004-09-02) * Seite 2, Zeile 22 - Seite 5, Zeile 24 * * Seite 8, Zeilen 9-15 * * Seite 10, Zeilen 19,20 * * Abbildungen 1,2 * ----- | 1-4,6,7, 11,18 | A61M G01N A61B G01D |
| A | WO 02/080764 A1 (RHODE ISLAND HOSPITAL A LIFESP [US]; JAY GREGORY D [US]; SUNER SELIM []) 17. Oktober 2002 (2002-10-17) * Seite 1, Zeile 20 - Seite 3, Zeile 9 * ----- | 16 | |

Der vorliegende Recherchenbericht wurde für alle Patentansprüche erstellt

| Recherchenort | Abschlußdatum der Recherche | Prüfer |
|---|---|---|
| München | 29. Januar 2007 | BICHLMAYER, K |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
..........................................................................
& : Mitglied der gleichen Patentfamilie, übereinstimmendes
    Dokument

**ANHANG ZUM EUROPÄISCHEN RECHERCHENBERICHT
ÜBER DIE EUROPÄISCHE PATENTANMELDUNG NR.**

EP 06 11 9406

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-01-2007

| Im Recherchenbericht angeführtes Patentdokument | | Datum der Veröffentlichung | Mitglied(er) der Patentfamilie | | | Datum der Veröffentlichung |
|---|---|---|---|---|---|---|
| EP 1574178 | A2 | 14-09-2005 | DE | 102004012447 | A1 | 06-10-2005 |
| DE 4014572 | A1 | 14-11-1991 | KEINE | | | |
| US 2003194894 | A1 | 16-10-2003 | AU | 2003223426 | A1 | 27-10-2003 |
| | | | EP | 1492584 | A1 | 05-01-2005 |
| | | | JP | 2006512101 | T | 13-04-2006 |
| | | | MX | PA04009856 | A | 07-12-2004 |
| | | | WO | 03086506 | A1 | 23-10-2003 |
| | | | US | 2006184087 | A1 | 17-08-2006 |
| DE 19848235 | C1 | 16-03-2000 | EP | 0995451 | A2 | 26-04-2000 |
| | | | ES | 2249866 | T3 | 01-04-2006 |
| | | | JP | 2000140101 | A | 23-05-2000 |
| | | | US | 6221040 | B1 | 24-04-2001 |
| WO 2004074822 | A | 02-09-2004 | DE | 10307454 | A1 | 09-09-2004 |
| | | | US | 2006176476 | A1 | 10-08-2006 |
| WO 02080764 | A1 | 17-10-2002 | CA | 2442921 | A1 | 17-10-2002 |
| | | | EP | 1383422 | A1 | 28-01-2004 |
| | | | JP | 2004535219 | T | 25-11-2004 |
| | | | US | 2003002722 | A1 | 02-01-2003 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1574178 A1
- WO 9924145 A1
- WO 0147581 A1
- DE 19848235 C1
- WO 0386506 A1